# EUROPÄISCHE PATENTSCHRIFT

(11) **EP 1 311 308 B1**
(45) Veröffentlichungstag und Bekanntmachung des Hinweises auf die Patenterteilung: **11.08.2004**
(21) Anmeldenummer: 01957671.9
(22) Anmeldetag: 13.08.2001
(51) Int. Cl.: A61L 27/12

(54) **KNOCHENERSATZMATERIAL**
BONE-REGENERATION MATERIAL
MATERIAU DE SUBSTITUTION DE L'OS

(30) Priorität: 22.08.2000 WO PCT/CH00/00443
(43) Veröffentlichungstag der Anmeldung: 21.05.2003
(73) Patentinhaber: SYNTHES AG Chur, 7002 Chur (CH)
(72) Erfinder: STOLL, Thierry, CH-2572 Sutz (CH)
(74) Vertreter: Lusuardi, Werther Giovanni, Dr.
(86) Internationale Anmeldenummer: PCT/CH2001/000494
(87) Internationale Veröffentlichungsnummer: WO 2002/015950

(56) Entgegenhaltungen:
- EP-A- 0 361 896
- EP-A- 0 470 393
- EP-A- 0 739 631
- DE-A- 3 834 944
- US-A- 5 755 787
- US-A- 5 876 452
- US-A- 6 027 742

## Beschreibung

Die Erfindung betrifft ein Verfahren zur Herstellung eines Knochenersatzmaterials gemäss dem Oberbegriff des Patentanspruchs 1, eine Vorrichtung zur Durchführung dieses Verfahrens gemäss dem Oberbegriff des Patentanspruchs 8, sowie ein Knochenersatzmaterial gemäss dem Oberbegriff des Patentanspruchs 10.

Aus der japanischen Patentanmeldung Nr. 60 142857 der SUMITOMO CEMENT CO. ist ein poröser Hydroxyapatit-Körper bekannt, welcher Wasser, wässerige Natriumchloridlösung, Blut und künstliches Blutplasma enthalten kann. Unklar bleibt bei diesem Dokument, ob es sich um einen offenporigen Körper handelt.

Aus der PCT/EP99/00684 sind Knochenersatzmaterialien bekannt, welche biokompatibel und osteokonduktiv sind. Die Osteokonduktion wird definiert als Wachstum von Osteoblasten und zwischenschichtfreie Knochenneubildung an der Oberfläche körpereigenen Materials oder von eingebrachten Fremdmaterialien, ohne dass diese unter dem Knochenanwuchs eine Veränderung erfahren.

Nachteilig bei diesen bekannten Materialien ist die fehlende Osteoinduktion. Unter Osteoinduktion versteht man einen im Prinzip unphysiologischen Eingriff in die Gewebeverteilung des Körpers. Osteoinduktion bedeutet Einleiten und Stimulieren von Knochenwachstum in einen Gewebebezirk, der kein Knochengewebe enthält, in diesem Zusammenhang die offenporige Struktur eines Biomaterials.

Aus der US 5.876452 ist es bekannt, ein bioaktives Mittel, bzw. Zellen durch Beigabe zu einer Polymerlösung und anschliessendem Entfernen des Polymer-Lösungsmittels in homogener Weise im Polymer zu verteilen, ohne dass sich dabei Poren bilden würden.
Aus der EP-A 0.470.303 ist ein Granulat bekannt, welches aus einer Vielzahl von einzelnen Kömern besteht, die selbst nicht porös sind. Durch Anwendung von Vakuum kann der zwischen den einzelnen Granulatkömen vorhandene freie Raum mit Blut gefüllt werden, ohne dass aber dadurch zufällig in den Granulatkömern vorhandene Poren ebenfalls mit Blut gefüllt würden, da dort kein zusätzliches Vakuum herrscht.
Aus der EP-A 0 361.896 ist schliesslich eine Prothese bekannt, welche den Knochen funktional ersetzt. Diese Prothese wird in offener Atmosphäre auf eine Glasfritte gelegt, um über deren Evakuation eine Suspension durch die Poren der Prothese saugen zu können. Dieses offene Verfahren ist aber naturgemäss nicht sehr effizient.

Hier will die Erfindung Abhilfe schaffen. Der Erfindung liegt das Problem zugrunde, ein einfaches und zuverlässiges Verfahren zur Herstellung eines Knochenersatzmateriats, insbesondere mit einer verbesserten Osteoinduktivität, zu schaffen. Eine weitere Aufgabe liegt in der Schaffung eines verbesserten Knochenersatzmaterials.

Die Erfindung löst die gestellte Aufgabe mit einem Verfahren, weiches die Merkmale des Anspruchs 1 aufweist.
Damit ist der Vorteil erzielbar, dass die in den Poren des biokompatiblen Körpers befindlich en Körperzellen (z.B. Blut, bzw. das darin enthaltene Fibrin) eine Netzstruktur für die in den porösen Körper neu einzuwachsenden Körperzellen bildet. Bei Verwendung von Blut wird das Einwachsen der Knochenzellen in den porösen Körper zudem noch durch die in den Blutplättchen vorhandenen Wachstumsfaktoren gefördert.

Ein Vorteil des erfindungsgemässen Verfahrens besteht darin, dass der poröse Körper nicht einfach in eine Suspension von Körperzellen (z.B. Blut) eingetaucht wird, um durch die Kapillarwirkung des Porensystems dieses zu füllen, oder auf eine evakuierbare Glasfritte gelegt wird, sondem aktiv ein Vakuum in den untereinander verbundenen Poren erzeugt wird, mittels dessen osteoinduktive und/oder osteogene Substanzen in fliessfähigem Zustand (z.B. in Form von Blut oder in Form einer wässerigen Suspension) homogen und bis ins Innerste des porösen Körpers eingesaugt werden können.

Die durch die Erfindung erreichten Vorteile sind im wesentlichen darin zu sehen, dass dank des erfindungsgemäss hergestellten Knochenersatzmaterials, dessen offenporige Struktur bei der Implantation in einen Defekt mit geeigneten Körperzellen, z.B. : Blut, Blutbestandteilen (oder knochenbildungsfördemde Wachstumsfaktoren), Knochenmark oder Knochenzellen (alle in für diesen Zweck geeigneter, fliessfähiger Form) gefüllt ist und osteoinduktive Eigenschaften besitzt. Das Blutgerinnsel im Innem der Struktur organisiert sich besser, ähnlich wie bei einer Frakturheilung, in ein kömiges, faserarmes, zell- und gefässreiches Bindegewebe: das Granulationsgewebe. Darin wandern diverse Zellen und beginnen mit dem Aufbau einer knorpeligen Matrix. Dieser Prozess schreitet fort, bis das ganze Granulationsgewebe durch Knorpel ersetzt ist und später kalzifiziert. Ohne diese mit der Erfindung erreichten Vorteile würde dieser ganze biologische Prozess durch die nur mit Luft gefüllten Poren verlangsamt oder verunmöglicht werden.

Statt Blut oder Blutbestandteilen (oder knochenbildungsfördernde Wachstumsfaktoren), welche an sich schon fliessfähig sind, können Körperzellen auch in einer körperverträglichen Flüssigkeit, vorzugsweise in einer wässerigen Lösung suspendiert werden, um sie in die Poren des offenporigen Körper aufzusaugen.

Bei einem bevorzugten Verfahren sind die Körperzellen aus den Gruppen: autologes Knochenmark, separierte konzentrierte Zellen aus autologem Knochenmark, kultivierte autologe Stammzellen, differenzierte autologe Stammzellen oder mesenchymale Zellen ausgewählt. Die Vorläuferzellen (Präkursoren) der Zellen des peripheren Blutes werden ständig von unreifen hämatopoetischen Zellen, den sogenannten Stammzellen, nachgebildet.
Vorteilhafterweise sind die Körperzellen autologer Natur. d.h. Spender und Empfänger der Körperzellen ist das gleiche Individuum.

Bei einer bevorzugten Ausführungsform der Erfindung sind den Körperzellen in fliessfähiger Form das Knochenwachstum fördemde Stoffe beigemischt, wie z.B.:
a) synthetische Wachstumsfaktoren;
b) rekombinante Wachstumsfaktoren, vorzugsweise β-Wachstumsfaktor (TGF-β) oder FGF-2 (Fibroblast Growth Factor);
c) natürliche oder synthetische Peptide;
d) blutplättchenabgeleiteter Wachstumsfaktor (PDFG);
e) insulinartiger Wachstumsfaktor (IGF);
f) Fibrin-äfs Endprodukt der -Blutgerinnurig;- öder
g) synthetisches Fibrin

Der für das erfindungsgemässe Verfahren verwendete, offenporige Körper besteht vorzugsweise aus einem bioresorbierbaren Material, vorzugsweise aus Hydroxylapatit oder Tricalciumphosphat. Die Porosität dieses Körpers beträgt zweckmässigerweise mindestens 25%, vorzugsweise mindestens 35%. Über 50% der Poren sollten zweckmässigerweise einen Durchmesser im Bereich von 200 bis 500 Mikron aufweisen. Die Verbindungen zwischen den einzelnen Poren sollten zweckmässigerweise einen Durchmesser im Bereich von 10 bis 300 Mikron, vorzugsweise von 200 bis 400 Mikron aufweisen.

Die Erfindung und Weiterbildungen der Erfindung werden im folgenden anhand eines Ausführungsbeispiels noch näher erläutert.

Es zeigt:

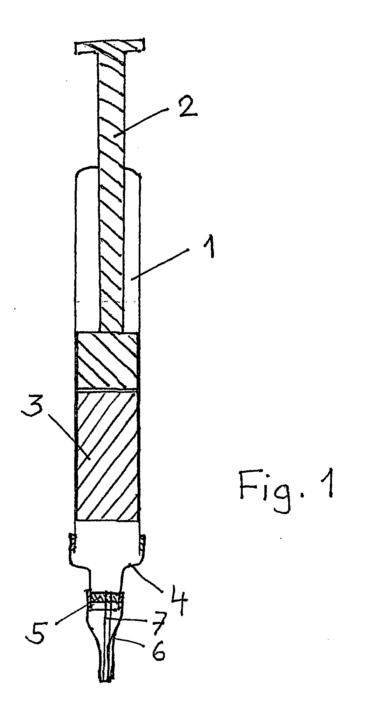

Fig.1 einen Schnitt durch eine Spritze zur Herstellung des erfindungsgemässen Knochenersatzmaterials.

Die in Fig. 1 dargestellte Spritze dient zur Herstellung des erfindungsgemässen Knochenersatzmaterials. Sie besteht aus einem Hohlzylinder 1, einem im Hohlzylinder beweglich angeordneten Kolben 2 und einem am vorderen Ende des Hohlzylinders 1 abschraubbaren Kopfteil 4. Dieser Kopfteil 4 besitzt eine Membran 5, welche mittels eines Adapters 6 mit Durchstechnadel 7 für die Blutentnahme durchgestochen wird. Beim Einsatz des Adapters 6 auf dem Kopfteil 4 wird die Adaptation eines Standart-Kanülensystem (z.B. Luer-System - Injektionsnadel, Katheter, Butterfly etc.) auf die Spritze möglich. Im Hohlzylinder 1 ist ein biokompatibler, offenporiger Körper 3 untergebracht, der den Hohlzylinder 1 im vorderen Abschnitt der Spritze abdichtet, so dass von der Spritze aufgesogenes Blut oder Blutbestandteile eines Patienten durch den Körper 3 fliessen müssen. Der Körper 3 besteht aus einem bioresorbierbaren Material, vorzugsweise aus Hydroxylapatit oder Tricalciumphosphat. Nach der Blutentnahme wird der Adapter 6 entfernt, somit ist die Membran 5 des Kopfteils 4 erneut dicht und der Inhalt des Hohlzylinders 1 der Spritze ist wieder luftdicht verschlossen. Durch das beim weiteren Zurückziehen des Kolbens 2 im Hohlzylinder 1 der Spritze entstehende Vakuum, durchströmt das Blut den gesamten Körper 3, dringt bis in dessen Zentrum und verdrängt die darin enthaltene Luft.

Nach erfolgter Imprägnierung des Körpers 3 mit Blut oder Blutbestandteilen (oder anderen Körperzellen in fliessfähigem Zustand) kann der Kopfteil 4 von der Spritze abgeschraubt werden, so dass der imprägnierte Körper 3 mittels des Kolbens 2 aus dem Hohlzylinder 1 gestossen werden kann. Der Körper 3 kann nun - gegebenenfalls nach erfolgter Anpassung seiner äusseren Form - in eine im Knochen des Patienten, dem das zur Imprägnierung benötigte Blut, bzw. anderen Körperzellen entnommen wurde, vorbereitete Bohrung oder in einen Knochendefekt eingesetzt werden. Durch die Offenporigkeit des Körpers 3 und das im Porensystem befindliche autologe Blut (oder Blutbestandteilen oder anderen Körperzellen) wird das Einwachsen von Körperzellen in den Körper 3 stark begünstigt.

## Patentansprüche

1. Verfahren zur Herstellung eines Knochenersatzmaterials, welches osteoinduktive und/oder osteogene Substanzen enthält,
**dadurch gekennzeichnet, dass**
A) die Poren eines biokompatiblen, offenporigen Körpers (3) einem Vakuum ausgesetzt werden;
B) die osteoinduktiven und/oder osteogenen Substanzen in fliessfähiger Form mittels des in den Poren des Körpers (3) erzeugten Vakuums in diese Poren aufgesaugt werden; wobei
C) das Verfahren in einer Spritze mit Hohlzylinder (1) und Kolben (2) durchgeführt wird, wobei die osteoinduktiven und/oder osteogenen Substanzen in fliessfähiger Form mit der Spritze aufgesogen und durch die Poren des im Hohlzylinder (1) der Spritze untergebrachten Körpers (3) hindurchgesogen werden.

2. Verfahren nach Anspruch 1, **dadurch gekennzeichnet, dass** die osteogenen Substanzen in Form von Körperzellen in fliessfähiger Form vorliegen, wobei die Körperzellen aus den Gruppen: a) autologes Knochenmark, b) separierte konzentrierte Zellen aus autologem Knochenmark, c) kultivierte autologe Stammzellen, d) differenzierte autologe Stammzell en oder e) mesenchymale Zellen ausgewählt sind.

3. Verfahren nach Anspruch 1, **dadurch gekennzeichnet, dass** die osteoinduktiven Substanzen in einer körperverträglichen Flüssigkeit, vorzugsweise in einer wässerigen Lösung enthalten sind.

4. Verfahren nach Anspruch 1, **dadurch gekennzeichnet, dass** die osteogenen Substanzen in einer körperverträglichen Lösung, vorzugsweise in einer wässerigen Lösung suspendiert sind.

5. Verfahren nach Anspruch 4, **dadurch gekennzeichnet, dass** die Körperzellen in fliessfähiger Form aus den Gruppen: A) Blut oder Blutbestandteile, B) Knochenmark oder C) Knochenzellen in fliessfähiger Form ausgewählt sind.

6. Verfahren nach einem der Ansprüche 1 bis 5, **dadurch gekennzeichnet, dass** die Körperzellen autologer Natur sind.

7. Verfahren nach einem der Ansprüche 1 bis 6, **dadurch gekennzeichnet, dass** die osteoinduktiven Substanzen aus folgenden Gruppen ausgewählt sind:
a) synthetische Wachstumsfaktoren;
b) rekombinante Wachstumsfaktoren, vorzugsweise β-Wachstumsfaktor (TGF-β) oder FGF-2 (Fibroblast Growth Factor);
c) natürliche oder synthetische Peptide;
d) blutplättchenabgeleiteter Wachstumsfaktor (PDFG);
e) insulinartiger Wachstumsfaktor (IGF);
f) Fibrin als Endprodukt der Blutgerinnung; oder
g) synthetisches Fibrin

8. Vorrichtung zur Durchführung des Verfahrens nach einem der Ansprüche 1 bis 7, **dadurch gekennzeichnet, dass** sie aus einer Spritze mit Hohlzylinder (1) und Kolben (2) besteht, wobei im Hohlzylinder (1) ein biokompatibler, offenporiger Körper (3) untergebracht ist, der den Hohlzylinder (1) mindestens abschnittsweise abdichtet, so dass von der Spritze aufgesogene osteoinduktive und/oder osteogene Substanzen in fliessfähiger Form durch die Poren des Körper (3) fliessen müssen.

9. Vorrichtung nach Anspruch 8, **dadurch gekennzeichnet, dass** die Spritze einen am vorderen Ende des Hohlzylinders (1) befestigbaren Kopfteil (4) mit Membran (5) aufweist, so dass von der Spritze aufgesogene, osteoinduktive und/oder osteogene Substanzen in fliessfähiger Form nach Verschliessen der Membran (5) und Zurückziehen des Kolbens (2) mittels Vakuum durch die Poren des Körper (3) fliessen müssen.

10. Knochenersatzmaterial herstellbar nach dem Verfahren gemäss einem der Ansprüche 1 bis 7.

11. Knochenersatzmaterial nach Anspruch 10, **dadurch gekennzeichnet, dass** der Körper (3) mindestens teilweise aus einem bioresorbierbaren Material, vorzugsweise aus Hydroxylapatit oder Tricalciumphosphat besteht.

12. Knochenersatzmaterial nach einem der Ansprüche 10 oder 11, **dadurch gekennzeichnet, dass** die Porosität des Körpers (3) mindestens 25%, vorzugsweise mindestens 35% beträgt.

13. Knochenersatzmaterial nach einem der Ansprüche 10 bis 12, **dadurch gekennzeichnet, dass** über 50% der Poren einen Durchmesser im Bereich von 200 bis 500 Mikron aufweisen.

14. Knochenersatzmaterial nach einem der Ansprüche 10 bis 13, **dadurch gekennzeichnet, dass** die Verbindung zwischen den einzelnen Poren einen Durchmesser im Bereich von 10 bis 300 Mikron, vorzugsweise von 200 bis 400 Mikron aufweisen.

## Claims

1. A method for preparing a bone replacement material, that comprises osteoinductive and /or osteogenic substances **characterised in that**
A) a biocompatible body (3) with fully interconnecting pores is exposed to a vacuum; and
B) that by means of the vacuum created in the pores of the body (3) the osteoinductive and / or osteogenic substances present in a free-flowing form are sucked into these pores; whereby
C) the method is carried out with the aid of a syringe including a hollow cylinder (1) and a piston (2), whereby the osteoinductive and/or osteogenic substances present in free-flowing form being absorbed by the syringe and being sucked through the pores of the body (3) accommodated within the hollow cylinder (1) of the syringe.

2. A method as claimed in claim 1, **characterised in that** the osteogenic substances are present in form of somatic cells in free-flowing form, whereby the somatic cells are selected from the following groups: a) autologous bone marrow, b) separated concentrated cells taken from autologous bone marrow, c) cultured autologous stem cells, d) differentiated autologous stem cells, or e) mesenchymal cells.

3. A method as claimed in claim 1, **characterised in that** the osteoinductive substances are contained in a biocompatible fluid, preferably in an aqueous solution.

4. A method as claimed in claim 1, **characterised in that** the osteoinductive substances are suspended in a biocompatible fluid, preferably in an aqueous solution.

5. A method as claimed in claim 4, **characterised in that** the somatic cells in free-flowing form are selected from the following groups: A) blood or blood constituents, B) bone marrow or C) bone cells in free-flowing form.

6. A method as claimed in any of the claims 1 to 5, **characterised in that** the somatic cells are of autologous nature.

7. A method as claimed in any of the claims 1 to 6, **characterised in that** the osteoinductive substances are selected from the following groups:
a) synthetic growth factors;
b) recombinant growth factors, preferably growth factor β (TGF-β) or FGF-2 (fibroblast growth factor);
c) natural or synthetic peptides;
d) platelet derived growth factor (PDGF);
e) insulin-like growth factor (IGF);
f) fibrin as the end product of blood coagulation; or
g) synthetic fibrin.

8. An apparatus for carrying out the method as claimed in any of the claims 1 to 7, **characterised in that** it consists of a syringe including a hollow cylinder (1) and a piston (2), a biocompatible body (3) with fully interconnecting pores being placed in such a way within the hollow cylinder (1) that it seals the hollow cylinder (1) at least over part of its length, so that the osteoinductive and/or osteogenic substances present in free-flowing form which are absorbed by the syringe are forced to flow through the pores of the body (3).

9. An apparatus according to claim 8, charcterised in that the syringe comprises a head portion (4) equipped with a membrane (5) at the front end of the hollow cylinder (1), such that osteoinductive and/or osteogenic substances present in free-flowing form are forced by means of vacuum to flow through the pores of the body (3) after sealing the membrane (5) and pulling back the piston (2).

10. A bone replacement material prepared according to the method as claimed in any of the claims 1 to 7.

11. A bone replacement material as claimed in claim, **characterised in that** the body (3) consists at least in part of a bioresorbable material, preferably of hydroxyapatite or tribasic calcium phosphate.

12. A bone replacement material as claimed in claim 10 or 11, **characterised in that** the porosity of the body (3) is at least 25 %, preferably at least 35 %.

13. A bone replacement material as claimed in any of the claims 10 to 12, **characterised in that** more than 50 % of the pores have a diameter ranging between 200 and 500 microns.

14. A bone replacement material as claimed in any of the claims 10 to 13, **characterised in that** the connections between the individual pores have a diameter ranging between 10 and 300 microns, preferably between 200 and 400 microns.

## Revendications

1. Procédé de fabrication d'un matériau de substitution de l'os contenant des substances ostéoinductive et/ou ostéogènes,
**caractérisé en ce que**
A) les pores d'un corps (3) biocompatible à pores ouverts sont soumis à un vide;
B) les substances ostéoinductive et/ou ostéogènes étant capable de s'écouler sont, au moyen du vide créé dans les pores du corps (3), aspirées dans lesdits pores;
C) le procédé étant appliqué à l'aide d'une seringue avec cylindre creux (1) et piston (2), les substances ostéoinductrices et/ou ostéogènes étant capable de s'écouler étant aspirées avec la seringue, passant ainsi à travers les pores du corps (3) placé dans le cylindre creux (1) de la seringue.

2. Procédé selon la revendication 1, **caractérisé en ce que** les substances ostéogènes se présentent sous forme de cellules somatiques étant capable de s'écouler, les cellules somatiques étant choisies dans les groupes : a) moelle osseuse autologue, b) cellules concentrées séparées issues de moelle osseuse autologue, c) cellules souches autologues cultivées, d) cellules souches autologues différenciées ou e) cellules mésenchymateuses.

3. Procédé selon la revendication 1, **caractérisé en ce que** les substances ostéoinductrices sont contenues dans un liquide supporté par l'organisme, de préférence dans une solution aqueuse.

4. Procédé selon la revendication 1, **caractérisé en ce que** les substances ostéogènes sont en suspension dans une solution supportée par l'organisme, de préférence dans une solution aqueuse.

5. Procédé selon la revendication 4, **caractérisé en ce que** les cellules somatiques étant capable de s'écouler sont choisies dans les groupes : A) sang ou constituants du sang, B) moelle osseuse ou C) cellules osseuses étant capable de s'écouler.

6. Procédé selon une des revendications 1 à 5, **caractérisé en ce que** les cellules somatiques sont de nature autologue.

7. Procédé selon une des revendications 1 à 6, **caractérisé en ce que** les substances ostéoinductrices sont choisies dans les groupes suivants :
a) facteurs de croissance synthétiques;
b) facteurs de croissance recombinants, de préférence facteur de croissance β (TGF-β) ou FGF-2 (Fibroblast Growth Factor - facteur de croissance fibroblastique);
c) peptides naturels ou synthétiques;
d) facteur de croissance dérivé des plaquettes (PDFG);
e) facteur de croissance analogue à l'insuline (IGF);
f) fibrine en tant que produit final de la coagulation du sang; ou
g) fibrine synthétique.

8. Dispositif d'application du procédé selon une des revendications 1 à 7, **caractérisé en ce qu'**il se compose d'une seringue avec cylindre creux (1) et piston (2), un corps (3) biocompatible à pores ouverts étant placé dans le cylindre creux (1) et rendant le cylindre creux (1) étanche au moins sur une partie, de sorte que les substances ostéoinductrices et/ou ostéogènes étant capable de s'écouler aspirées par la seringue traversent obligatoirement les pores du corps (3).

9. Dispositif selon la revendication 8, **caractérisé en ce que** la seringue présente une tête (4) pouvant être fixée sur l'extrémité avant du cylindre creux (1) et munie d'une membrane (5), de sorte que les substances ostéoinductrices et/ou ostéogènes étant capable de s'écouler aspirées par la seringue traversent obligatoirement les pores du corps (3) à l'aide du vide, lorsque la membrane (5) est fermée et que l'on tire le piston (2).

10. Matériau de substitution de l'os pouvant être fabriqué selon le procédé conformément à l'une des revendications 1 à 7.

11. Matériau de substitution de l'os selon la revendication 10, **caractérisé en ce que** le corps (3) se compose au moins en partie d'un matériau biorésorbable, de préférence de l'hydroxylapatite ou du phosphate tricalcique.

12. Matériau de substitution de l'os selon une des revendications 10 ou 11, **caractérisé en ce que** la porosité du corps (3) est d'au moins 25%, de préférence d'au moins 35%.

13. Matériau de substitution de l'os selon une des revendications 10 à 12, **caractérisé en ce que** plus de 50% des pores présentent un diamètre compris entre 200 et 500 microns.

14. Matériau de substitution de l'os selon une des revendications 10 à 13, **caractérisé en ce que** les liaisons entre chaque pore présentent un diamètre compris entre 10 et 300 microns, de préférence entre 200 et 400 microns.
